# FASCICULE DE BREVET EUROPEEN

(11) **EP 3 969 084 B1**
(45) Date de publication et mention de la délivrance du brevet: **15.02.2023**
(21) Numéro de dépôt: 20737233.5
(22) Date de dépôt: 14.05.2020
(51) Int. Cl.: A61M 11/00, A61M 15/00, A61M 15/08, A61M 31/00, A61M 35/00, B05B 7/24, B05B 9/08

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 17.05.2019 FR 1905215
(43) Date de publication de la demande: 23.03.2022
(73) Titulaire: APTAR France SAS, 27110 Le Neubourg (FR)
(72) Inventeur: ADAM, Fabien, 27930 Aviron (FR); BAILLET, Matthieu, 76000 Rouen (FR)
(74) Mandataire: CAPRI
(86) Numéro de dépôt international: PCT/FR2020/050797
(87) Numéro de publication internationale: WO 2020/234527

(56) Documents cités:
- WO-A1-00/47332
- WO-A1-2004/026379
- WO-A1-2004/050508
- WO-A1-2012/160157
- WO-A1-2016/123110
- WO-A2-03/082702
- DE-U1- 20 107 507
- US-A1- 2007 240 712
- US-A1- 2019 015 613
- US-A1- 2019 022 316

## Description

La présente invention concerne un dispositif de distribution de produit fluide, et plus particulièrement un dispositif du type combidose, c'est-à-dire dans lequel deux produits fluides sont mélangés et distribués simultanément au moment de l'actionnement.

Des distributeurs du type combidose ont été développés pour de nombreuses applications, notamment dans le domaine de la pharmacie. Ces distributeurs sont utilisés notamment dans le domaine de la distribution nasale. Ces dispositifs comportent généralement deux réservoirs séparés contenant chacun un produit fluide différent, les produits étant mélangés avant ou au cours de la distribution pour être distribués ensemble. Les réservoirs peuvent contenir chacun une seule ou plusieurs doses, selon le type de dispositifs. Un inconvénient des dispositifs avec des réservoirs multidoses est la nécessité de prévoir des moyens de dosage pour mesurer une dose de chaque produit fluide à chaque actionnement. Ces moyens de dosage rendent le dispositif complexe à fabriquer et à assembler. De plus, il peut y avoir des risques de contamination des produits fluides contenus dans chaque réservoir, notamment entre deux actionnements. Un inconvénient des dispositifs avec des réservoirs unidoses est qu'ils ne sont utilisables qu'une seule fois, l'ensemble du dispositif devant être jeté après usage.

De plus, selon la nature des produits fluides, particulièrement lorsqu'il s'agit de médicaments, les conditions de remplissage et de stockage du produit peuvent être assez contraignantes. Ainsi, de nombreux produits fluides du domaine pharmaceutique nécessitent un remplissage en zone stérile et/ou un stockage en chambre froide. Dans les dispositifs de distribution existants, les produits sont généralement remplis après assemblage complet du dispositif, ce qui implique des machines de remplissage spécialement adaptées aux dispositifs en question, ces machines devant bien entendu être dans des zones stériles. Après remplissage, le dispositif dans son ensemble doit être stocké en chambre froide. Etant donné les coûts très élevés des surfaces industrielles en zones stériles et des volumes en chambres froides, l'utilisation de machines de remplissage spécifiques s'avère être un inconvénient du point de vue économique, et il en est de même en ce qui concerne le stockage en chambre froide.

Les documents US2007240712, WO03082702, WO2004026379, WO2004050508, WO0047332, US2019015613, DE20107507, WO2012160157, WO2016123110 et US2019022316 décrivent des dispositifs de l'état de la technique. Le document WO 2004/026379 A1, par exemple, décrit un dispositif de pulvérisation de produit fluide, comportant un corps pourvu d'un orifice de pulvérisation, un réservoir contenant le produit fluide à pulvériser, des moyens de pulvérisation pour pulvériser en une ou plusieurs doses le produit contenu dans le réservoir, et des moyens d'actionnement pour actionner lesdits moyens de pulvérisation, ledit réservoir étant fermé de manière étanche avant le premier actionnement du dispositif de pulvérisation, le corps comportant des moyens d'ouverture du réservoir adaptés à ouvrir ledit réservoir lors de l'actionnement du dispositif, ledit réservoir formant une unité étanche séparée dudit corps, ledit réservoir étant rempli avec le produit fluide et fermé hermétiquement avant son assemblage dans ledit corps, et ledit corps comportant des moyens de réception de réservoir et des moyens d'accès latéral pour assembler latéralement et fixer ledit réservoir rempli dans ledit corps.

La présente invention a pour but de fournir un dispositif de distribution de produit fluide qui ne reproduit pas les inconvénients susmentionnés.

Ainsi, la présente invention a pour but de fournir un dispositif de distribution de produit fluide du type combidose qui soit réutilisable.

La présente invention a également pour but de fournir un tel dispositif de distribution de produit fluide pour lequel les surfaces et volumes de zones stériles lors du remplissage et de chambres froides lors du stockage sont minimales.

La présente invention a également pour but de fournir un tel dispositif de distribution de produit fluide qui soit simple et peu coûteux à fabriquer et à assembler, et fiable dans son utilisation.

La présente invention a donc pour objet un dispositif de distribution de produit fluide tel que décrit dans la revendication 1. Des modes de réalisation avantageux sont décrits dans les revendications dépendantes.

D'autres caractéristiques et avantages de la présente invention apparaîtront plus clairement au cours de la description détaillée suivante, faite en référence aux dessins joints, donnés à titre d'exemples non limitatifs, et sur lesquels :
La figure 1 est une vue schématique en perspective d'un dispositif de distribution de produit fluide selon un mode de réalisation avantageux;
La figure 2 est une vue schématique en section transversale d'un dispositif de distribution de produit fluide selon un mode de réalisation avantageux, avant insertion des réservoirs ;
La figure 3 est une vue similaire à celle de la figure 2, un réservoir étant fixé à l'intérieur du dispositif et l'autre réservoir étant en cours d'insertion ;
La figure 4 est une vue similaire à celle de la figure 3, en début d'actionnement du dispositif ; et
La figure 5 est une vue similaire à celle de la figure 4, en fin d'actionnement du dispositif.

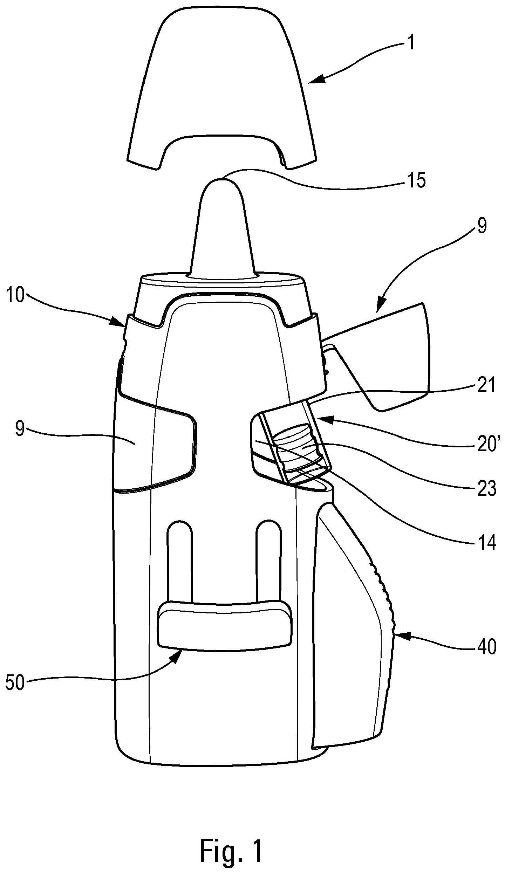

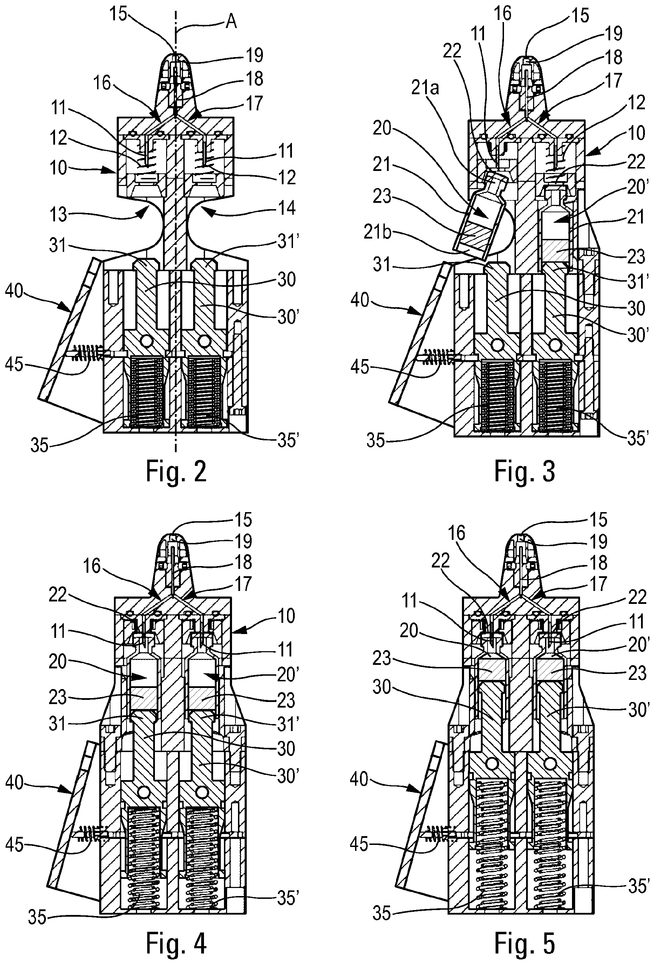

Dans la description, les termes "supérieur", "inférieur", "haut" et "bas" se réfèrent à la position droite du dispositif représentée sur les figures 1 à 5. Les termes "axial" et "radial" se réfèrent à l'axe central vertical A de la pompe, représenté sur la figure 2. Les termes "proximal" et "distal" se réfère à l'orifice de distribution.

En référence aux figures, il est représenté un dispositif de distribution du type combidose. Ce dispositif comporte un corps 10 qui est pourvu d'un orifice de distribution 15. Dans l'exemple représenté, l'orifice de distribution 15 est formé à l'extrémité axiale d'une tête de distribution nasale, qui est la mise en oeuvre préférée de l'invention. Il est néanmoins à noter qu'une distribution buccale, auriculaire ou topique pourrait aussi être envisagée. Un capot de protection amovible 1, visible sur la figure 1, peut être prévu pour protéger l'orifice de distribution 15 entre deux actionnements. Le corps 10 reçoit deux réservoirs 20, 20', chacun contenant une dose unique d'un produit fluide. Généralement, ces deux produits fluides sont différents, mais ils pourraient aussi être identiques ou similaires, par exemple si après chaque actionnement on utilise des réservoirs de nettoyage comme cela sera décrit ultérieurement.

Les deux réservoirs 20, 20' sont avantageusement identiques. Un seul réservoir sera donc décrit plus en détail ci-après. Chaque réservoir forme une unité étanche séparée du reste du dispositif, et est avantageusement constituée d'un tube creux 21, comportant une première ouverture axiale 21a et une seconde ouverture axiale 21b. La première ouverture axiale 21a peut être formée dans une partie de col dudit tube creux 21. A l'état assemblé des réservoirs dans le corps 10, la première ouverture 21a forme l'ouverture proximale et la seconde ouverture axiale 21b forme l'ouverture distale. La première ouverture axiale 21a est bouchée par une première fermeture étanche 22, et la seconde ouverture axiale 21b est bouchée par une seconde fermeture étanche 23. Le tube creux 21 peut être en verre ou tout autre matériau approprié. La première fermeture étanche 22 peut être une membrane et la seconde fermeture étanche 23 peut être un bouchon 23, avantageusement réalisé en matériau élastomère. En variante, la première fermeture étanche 23 pourrait aussi être un bouchon 23, avantageusement réalisé en matériau élastomère.

Ce type de réservoir, qui ne contient qu'une seule dose de produit, est de très petite dimension, ce qui limite les surfaces de zone stérile pour le remplissage et les volumes de chambre froide pour le stockage de ces réservoirs après leur remplissage.

Le corps 10 comporte des moyens d'ouverture 11 associés à chaque réservoir, ces moyens pouvant par exemple être formés par des moyens de percement, tel que des aiguilles. Chaque aiguille 11 est destinée à percer la première fermeture étanche 22 d'un réservoir 20, 20' respectif lors de l'actionnement du dispositif. De préférence, chaque aiguille 11 est fixe par rapport au corps 10 et reliée à l'orifice de distribution 15 via un canal d'expulsion respectif 16, 17. De préférence, comme visible sur les figures, les canaux d'expulsion 16, 17 des deux aiguilles 11 se rejoignent en amont de l'orifice de distribution 15 pour former un canal d'expulsion unique 18 menant audit orifice de distribution 15. Avantageusement, un profil de pulvérisation 19 est prévu juste en amont dudit orifice de distribution 15, de manière bien connue.

Le dispositif de distribution comporte en outre des moyens de distribution 30, 30' et des moyens d'actionnement 40 destinés à actionner lesdits moyens de distribution. Les moyens de distribution comportent une tige respective 30, 30' pour chaque réservoir 20, 20', lesdites tiges étant déplaçable axialement, pour, lors de l'actionnement, coopérer chacune avec le bouchon 23 d'un réservoir respectif pour le déplacer axialement à l'intérieur de son réservoir respectif. Lesdits bouchons 23 se transforment ainsi en pistons qui vont coulisser dans les tubes creux 21 pour expulser les doses des deux produits fluides à travers les aiguilles 11 en direction de l'orifice de distribution 15. Chaque tige 30, 30' est associée à un ressort d'actionnement 35, 35' respectif adapté à exercer la force axiale pour réaliser la distribution du contenu des réservoirs 20, 20'. En variante, on pourrait envisager une seule tige 30 associée à un seul ressort d'actionnement 35, ladite tige pouvant être pourvue à son extrémité proximale de deux branches parallèles dont chacune coopérerait avec un réservoir 20, 20' respectif.

Dans l'exemple représenté, les ressorts d'actionnements 35, 35' sont disposés entre ledit corps 10 et des extrémités axiales distales desdites tiges 30, 30'.

Lesdites tiges sont associées à un tiroir axial 50 s'étendant hors dudit corps 10, comme visible sur la figure 1. Ce tiroir axial 50 est déplaçable axialement par rapport audit corps 10, ensemble avec lesdites tiges 30, 30', entre une position non chargée et une position chargée. Ainsi, lorsque l'utilisateur déplace ledit tiroir axial 50 vers le bas vers sa position chargée, il charge lesdits ressorts d'actionnement 35, 35'. Lors de l'actionnement, les tiges 30, 30' ensemble avec le tiroir axial 50 sont déplacées axialement vers le haut par lesdits ressorts d'actionnement 35, 35', ramenant le tiroir axial 50 dans sa position non chargée.

Avantageusement, lesdites tiges 30, 30' et/ou ledit tiroir axial 50 comportent des moyens de blocage en position chargée, lesdits moyens de blocage étant libérés par les moyens d'actionnement 40 lors de l'actionnement du dispositif. Ces moyens de blocage peuvent par exemple comporter des moyens d'encliquetage qui viennent bloquer les tiges 30, 30' en position chargée et qui sont désencliquetés lorsque les moyens d'actionnement 40 sont actionnés.

De préférence, les moyens d'actionnement 40 comportent un élément d'actionnement latéral, c'est-à-dire un élément qui se déplace dans une direction différente de la direction de déplacement des moyens de distribution. Dans l'exemple représenté, l'élément d'actionnement latéral est avantageusement un levier latéral 40, déplaçable, notamment pivotant, par rapport audit corps 10 entre une position de repos, visible sur les figures 1 et 2, et une position d'actionnement, visible sur les figures 3 et 4. Un ressort de rappel 45 sollicite de préférence ledit levier latéral 40 vers sa position de repos.

Chaque aiguille 11 est de préférence associée à un ressort d'aiguille 12 respectif adapté à coopérer avec la première ouverture 21a de chaque réservoir, pour solliciter ledit réservoir en éloignement de l'aiguille 11 respective. Ceci permet de garantir que l'ouverture des réservoirs par percement des premières fermetures étanches 22 ne se fera que lors de l'actionnement du dispositif. De manière évidente, la force desdits ressorts d'aiguille 12 est inférieure à celle desdits ressorts d'actionnement 35, 35'.

Le corps 10 comporte des moyens de réception 13, 14 des réservoirs qui forment des moyens d'accès latéral. Ces moyens de réception permettent un chargement des réservoirs 20, 20' à l'intérieur du corps 10 de manière latérale. L'assemblage des réservoirs 20, 20' à l'intérieur du corps 10 peut être réalisé juste avant l'utilisation du dispositif. Avantageusement, les moyens de réception 13, 14 comportent chacun une fenêtre respective réalisée dans une paroi latérale du corps 10. Les figures montrent un corps 10 comportant deux fenêtres latérales diamétralement opposées. Les moyens de réception peuvent comporter des moyens de fixation, par exemple des moyens d'encliquetage, ou tout autre moyen approprié, qui permettent de maintenir les réservoirs 20, 20' à l'intérieur du corps 10. Dans l'exemple représenté sur les figures, l'ouverture distale 21b de chaque réservoir vient se positionner sur l'extrémité axiale proximale 31, 31' des tiges 30, 30', en étant maintenu en position par les ressorts d'aiguille 12 qui pousse les réservoirs axialement vers le bas. Dans cette mise en oeuvre, les bouchons 23 qui obturent les ouvertures distales 21b des réservoirs sont légèrement décalés à l'intérieur des corps creux 21, pour permettre auxdites extrémités axiales proximales 31, 31' des tiges 30, 30' de légèrement pénétrer dans lesdits corps creux. Bien entendu, d'autres moyens de fixation ou de butée complémentaires peuvent être prévus pour favoriser une bonne fixation de ces réservoirs 20, 20' dans le corps 10. Eventuellement, un couvercle amovible 9 respectif peut être prévu au niveau des moyens de réception 13, 14, ces couvercles 9 pouvant être réalisés d'une façon quelconque souhaitable. La figure 1 illustre un exemple de réalisation possible, dans lequel les deux couvercles 9 sont pivotants sur le corps 10.

Le chargement latéral des réservoirs dans le corps 10 permet donc de réaliser des réservoirs séparés, de les remplir et de les boucher de manière hermétique puis de les stocker indépendamment du reste du dispositif de distribution. Le reste du dispositif peut de son côté être assemblé dans une zone non stérile et stocké dans un local qui n'est pas une chambre froide. Ce n'est que très peu avant l'utilisation du dispositif que les réservoirs peuvent être assemblés dans le corps 10, le contenu des réservoirs restant protégé par les fermetures étanches 22, 23 jusqu'à l'actionnement du dispositif. Le chargement latéral des réservoirs présente l'avantage de pouvoir utiliser un actionnement latéral avec des moyens de distribution se déplaçant de manière axiale, et de pouvoir assembler la totalité du dispositif, indépendamment des réservoirs. L'assemblage du dispositif en est simplifié, et le dispositif lui-même est également simplifié, en économisant notamment une pièce, généralement nécessaire pour pré-assembler les réservoirs lorsque ceux-ci doivent être assemblés de manière axiale à l'intérieur du corps. La présente invention permet donc de réaliser un dispositif de distribution de produit fluide moins cher, de fonctionnement fiable, et pour lequel des économies importantes peuvent être réalisées lors du remplissage et du stockage.

Le fonctionnement du dispositif représenté sur les dessins est le suivant: avec le levier axial 50 bloqué en position chargée, l'utilisateur peut insérer un premier réservoir 20 contenant une dose d'un premier produit fluide dans le corps 10 à travers la fenêtre 13 du corps. Il peut ensuite insérer un second réservoir 20' contenant une dose d'un second produit fluide dans le corps 10 à travers la fenêtre 14 du corps. Les ressorts d'aiguille sollicitent lesdits réservoirs 20, 20' axialement vers le bas, en éloignement des aiguilles 11, pour garantir l'intégrité du contenu desdits réservoirs. Le dispositif est alors prêt à être actionné.

L'utilisateur appuie alors sur le levier latéral 40, ce qui va déplacer celui-ci vers sa position d'actionnement. Ce déplacement du levier latéral 40 va libérer les moyens de blocage, et ainsi les tiges 30, 30' vont se déplacer axialement vers le haut sous l'effet des ressorts d'actionnement 35, 35' comprimés. La force nécessaire pour déplacer les bouchons 23 dans les corps creux 21 des réservoirs 20, 20' étant supérieure à la force exercée par les ressorts d'aiguille 12 sur les réservoirs 20, 20', le déplacement axial des tiges 30, 30' provoque d'abord le déplacement des réservoirs 20, 20' axialement vers le haut, provoquant le percement des membranes 22 par les aiguilles 11. Ensuite, les bouchons 23 sont déplacés dans les réservoirs 20, 20' pour expulser les produits fluides contenus dans les deux réservoirs. Les deux produits fluides sont donc expulsés simultanément, chacun à travers son aiguille 11 respective, et ils vont se mélanger dans le canal d'expulsion unique 18, ledit mélange étant ensuite pulvérisé à travers l'orifice de distribution 15.

Après la distribution des deux produits fluides mélangés, l'utilisateur ramène le tiroir axial 50 vers sa position chargée, rechargeant ainsi les ressorts d'actionnement 35, 35', jusqu'à ce que les tiges 30, 30' et le tiroir axial 50 soient bloqués dans la position chargée par les moyens de blocage. Il peut alors ouvrir les deux couvercles 9 et retirer les deux réservoirs 20, 20' vides, par exemple en les repoussant manuellement axialement vers le haut contre la force des ressorts d'aiguille 12, pour désengager l'ouverture distale 21b de chaque réservoir de l'extrémité supérieure 31, 31' des tiges 30, 30'. Après retrait des deux réservoirs 20, 20' du corps 10, l'utilisateur peut éventuellement laver ou nettoyer le dispositif, et notamment les aiguilles 11 et l'orifice de distribution, puis stocker le dispositif en vue d'une prochaine utilisation avec deux nouveaux réservoirs pleins.

Avantageusement, le nettoyage est réalisé au moyens de réservoirs de nettoyage, identiques aux réservoirs 20, 20' mais contenant un produit fluide nettoyant et/ou décontaminant. Ceci permet de nettoyer les aiguilles 11, les canaux d'expulsion 16, 17, 18, le profil de pulvérisation 19 et l'orifice de distribution 15. On assure ainsi que la prochaine utilisation avec des produits actifs n'est pas polluée par la précédente, en évitant notamment tout risque de surdose ou d'incompatibilité si les réservoirs suivants contiennent des produits différents. Le cycle d'actionnement pour un tel nettoyage est identique au cycle d'actionnement décrit précédemment, sauf évidemment que le produit fluide de nettoyage n'est pas distribué dans le nez, la bouche, les oreilles ou sur la peau de l'utilisateur.

La présente invention peut notamment mais pas exclusivement être utilisée pour traiter les maladies de Parkinson et de Huntington, la migraine, la vaccination nasal, l'overdose, la crise cardiaque.

Bien que l'invention ait été décrite en référence à un exemple de réalisation particulier de celle-ci, il est entendu qu'un homme du métier peut y apporter toutes modifications, sans sortir du cadre de la présente invention tel que défini par les revendications annexées.

## Revendications

1. Dispositif de distribution de produit fluide, comportant un corps (10) pourvu d'un orifice de distribution (15), deux réservoirs amovibles (20, 20') contenant chacun une dose unique de produit fluide à distribuer, des moyens de distribution (30, 30'; 35, 35') pour distribuer lesdits produits fluides contenu dans lesdits réservoirs (20, 20'), et des moyens d'actionnement (40) pour actionner lesdits moyens de distribution (30, 30' ; 35, 35'), lesdits réservoirs (20, 20') étant fermés de manière étanche avant l'actionnement du dispositif de distribution, le corps (10) comportant des moyens d'ouverture (11) de chaque réservoir (20, 20'), lesdits moyens d'ouverture étant adaptés à ouvrir lesdits réservoirs (20, 20') lors de l'actionnement du dispositif, lesdits réservoirs (20, 20') formant chacun une unité étanche séparée dudit corps (10), chaque réservoir (20, 20') étant rempli avec un produit fluide respectif et fermé hermétiquement avant son assemblage dans ledit corps (10), chaque réservoir (20, 20') comportant un tube creux (21), comportant une première ouverture axiale (21a) proximale et une seconde ouverture axiale (21b) distale, ladite première ouverture axiale (21a) étant bouchée par une première fermeture étanche (22), et ladite seconde ouverture axiale (21b) étant bouchée par une seconde fermeture étanche (23), ledit corps (10) comportant des moyens de réception (13, 14) pour assembler latéralement lesdits réservoirs (20, 20') dans ledit corps (10), lesdits moyens de distribution (30, 30'; 35, 35') comportant une tige associée à chaque réservoir, lesdites tiges étant déplaçables axialement et coopérant avec la seconde fermeture étanche (23) de chaque réservoir (20, 20'), chaque tige (30, 30') étant associée à un ressort d'actionnement (35, 35') adapter à déplacer axialement lesdites tiges lors de l'actionnement, lesdites tiges (30, 30') étant
solidaires d'un tiroir axial (50) s'étendant hors dudit corps (10), ledit tiroir axial étant déplaçable axialement par rapport audit corps (10) entre une position chargée, dans laquelle lesdits ressorts d'actionnement (35, 35') sont chargés et une position non chargée après actionnement, l'utilisateur ramenant manuellement ledit tiroir en position chargée après chaque actionnement pour recharger lesdits ressorts d'actionnement (35, 35').

2. Dispositif selon la revendication 1, dans lequel lesdits moyens de réception comprennent une fenêtre (13, 14) prévue pour chaque réservoir (20, 20') dans une paroi latérale dudit corps (10).

3. Dispositif selon la revendication 1 ou 2, dans lequel ladite première fermeture étanche (22) est une membrane.

4. Dispositif selon l'une quelconque des revendications précédentes, dans lequel ladite seconde fermeture étanche (23) est un bouchon, notamment en élastomère.

5. Dispositif selon l'une quelconque des revendications précédentes, dans lequel chaque réservoir (20, 20') contient un produit fluide différent, les deux produits fluides différents se mélangeant lors de l'actionnement pour être distribués ensemble.

6. Dispositif selon l'une quelconque des revendications 1 à 4, dans lequel chaque réservoir contient un produit fluide de nettoyage et/ou décontaminant pour former un réservoir de nettoyage.

7. Dispositif selon l'une quelconque des revendications précédentes, dans lequel lesdits moyens d'actionnement (40) comportent un élément d'actionnement latéral déplaçable dans une direction différente de la direction de déplacement desdits moyens de distribution (30, 30').

8. Dispositif selon l'une quelconque des revendications précédentes, dans lequel lesdits moyens d'ouverture (11) des réservoirs comportent des moyens de percement, tels qu'une aiguille, de ladite première fermeture étanche (22) de chaque réservoir (20, 20').

9. Dispositif selon la revendication 8, dans lequel chaque aiguille (11) est associée à un ressort d'aiguille (12) qui sollicite un réservoir respectif (20, 20'), lorsqu'il est assemblé dans ledit corps (10), en éloignement de ladite aiguille (11).

10. Dispositif selon l'une quelconque des revendications précédentes, dans lequel ledit corps (10), lesdits moyens de distribution (30, 30'; 35, 35'), lesdits moyens d'actionnement (40) et lesdits moyens d'ouverture (11) sont assemblés pour former une unité, lesdits réservoirs (20, 20'), après remplissage et bouchage, étant assemblés dans cette unité.

11. Dispositif selon l'une quelconque des revendications précédentes, dans lequel lesdits moyens de réception (13, 14) comportent un couvercle amovible (9).

## Patentansprüche

1. Abgabevorrichtung für ein Fluidprodukt, umfassend einen mit einer Abgabeöffnung (15) versehenen Körper (10), zwei abnehmbare Vorratsbehälter (20, 20'), die jeweils eine Einzeldosis des abzugebenden Fluidprodukts enthalten, Abgabemittel (30, 30'; 35, 35') zur Abgabe der in den Vorratsbehältern (20, 20') enthaltenen Fluidprodukte und Betätigungsmittel (40) zum Betätigen der Abgabemittel (30, 30' ; 35, 35'), wobei die Vorratsbehälter (20, 20') vor Betätigung der Abgabevorrichtung dicht verschlossen sind, wobei der Körper (10) Öffnungsmittel (11) für jeden Vorratsbehälter (20, 20') umfasst, wobei die Öffnungsmittel geeignet sind, die Vorratsbehälter (20, 20') bei Betätigung der Vorrichtung zu öffnen, wobei die Vorratsbehälter (20, 20') jeweils eine vom Körper (10) getrennte abgedichtete Einheit bilden, wobei jeder Vorratsbehälter (20, 20') vor seiner Anbringung im Körper (10) mit einem jeweiligen Fluidprodukt gefüllt und hermetisch verschlossen wird, wobei jeder Vorratsbehälter (20, 20') ein Hohlrohr (21) umfasst, das eine erste proximale axiale Öffnung (21a) und eine zweite distale axiale Öffnung (21b) umfasst, wobei die erste axiale Öffnung (21a) mit einem ersten dichten Verschluss (22) verschlossen ist und die zweite axiale Öffnung (21b) mit einem zweiten dichten Verschluss (23) verschlossen ist, wobei der Körper (10) Aufnahmemittel (13, 14) zur seitlichen Montage der Vorratsbehälter (20, 20') in dem Körper (10) umfasst, wobei die Abgabemittel (30, 30'; 35, 35') eine jedem Vorratsbehälter zugeordnete Stange umfassen, wobei die Stangen axial verschiebbar sind und mit dem zweiten dichten Verschluss (23) jedes Vorratsbehälters (20, 20') zusammenwirken, wobei jede Stange (30, 30') einer Betätigungsfeder (35, 35') zugeordnet ist, die geeignet ist, die Stangen bei Betätigung axial zu verschieben, wobei die Stangen (30, 30') fest mit einem aus dem Körper (10) heraus verlaufenden Axialschieber (50) verbunden sind, wobei der Axialschieber in Bezug auf den Körper (10) zwischen einer belasteten Position, in der die Betätigungsfedern (35, 35') belastet sind, und einer unbelasteten Position nach Betätigung axial verschiebbar ist, wobei der Benutzer den Schieber nach jeder Betätigung manuell in die belastete Position zurückführt, um die Betätigungsfedern (35, 35') neu zu belasten.

2. Vorrichtung nach Anspruch 1, bei der die Aufnahmemittel ein Fenster (13, 14) umfassen, das für jeden Vorratsbehälter (20, 20') in einer Seitenwand des Körpers (10) vorgesehen ist.

3. Vorrichtung nach Anspruch 1 oder 2, bei der es sich bei dem ersten dichten Verschluss (22) um eine Membran handelt.

4. Vorrichtung nach einem der vorhergehenden Ansprüche, bei der es sich bei dem zweiten dichten Verschluss (23) um einen Stopfen, insbesondere aus Elastomer, handelt.

5. Vorrichtung nach einem der vorhergehenden Ansprüche, bei der jeder Vorratsbehälter (20, 20') ein anderes Fluidprodukt enthält, wobei bei Betätigung eine Vermischung der beiden unterschiedlichen Fluidprodukte zu deren gemeinsamer Abgabe erfolgt.

6. Vorrichtung nach einem der Ansprüche 1 bis 4, bei der jeder Vorratsbehälter ein Fluidprodukt zur Reinigung und/oder Dekontamination enthält und so einen Reinigungstank bildet.

7. Vorrichtung nach einem der vorhergehenden Ansprüche, bei der die Betätigungsmittel (40) ein seitliches Betätigungselement umfassen, das in einer anderen Richtung als die Bewegungsrichtung der Abgabemittel (30, 30') bewegbar ist.

8. Vorrichtung nach einem der vorhergehenden Ansprüche, bei der die Öffnungsmittel (11) für die Vorratsbehälter Mittel zum Durchstechen, beispielsweise eine Nadel, des ersten dichten Verschlusses (22) jedes Vorratsbehälters (20, 20') umfassen.

9. Vorrichtung nach Anspruch 8, bei der jede Nadel (11) mit einer Nadelfeder (12) verbunden ist, die einen jeweiligen Vorratsbehälter (20, 20') nach dessen Montage im Körper (10) von der Nadel (11) weg vorspannt.

10. Vorrichtung nach einem der vorhergehenden Ansprüche, bei der der Körper (10), die Abgabemittel (30, 30'; 35, 35'), die Betätigungsmittel (40) und die Öffnungsmittel (11) zu einer Einheit zusammengebaut sind, wobei die Vorratsbehälter (20, 20') nach deren Befüllung und Verschluß in dieser Einheit montiert werden.

11. Vorrichtung nach einem der vorhergehenden Ansprüche, bei der die Aufnahmemittel (13, 14) einen abnehmbaren Deckel (9) umfassen.

## Claims

1. A device for dispensing a fluid product, comprising a body (10) provided with a dispensing orifice (15), two removable reservoirs (20, 20') each containing a single dose of fluid product to be dispensed, dispensing means (30, 30'; 35, 35') for dispensing said fluid products contained in said reservoirs (20, 20'), and actuating means (40) for actuating said dispensing means (30, 30'; 35, 35'), said reservoirs (20, 20') being closed in a sealed manner before the dispensing device is actuated, the body (10) comprising means (11) for opening each reservoir (20, 20'), said opening means being adapted to open said reservoirs (20, 20') during actuation of the device, said reservoirs (20, 20') each forming a sealed unit which is separate from said body (10), each reservoir (20, 20') being filled with a respective fluid product and hermetically sealed before it is installed in said body (10), each reservoir (20, 20') comprising a hollow tube (21), comprising a first proximal axial opening (21a) and a second distal axial opening (21b), said first axial opening (21a) being plugged by a first sealed closure (22), and said second axial opening (21b) being plugged by a second sealed closure (23),said body (10) comprising receiving means (13, 14) for lateral installation of said reservoirs (20, 20') in said body (10), said dispensing means (30, 30'; 35, 35') comprising a rod associated with each reservoir, said rods being axially displaceable and cooperating with the second sealed closure (23) of each reservoir (20, 20'), each rod (30, 30') being associated with an actuating spring (35, 35') which is adapted for axially displacing said rods during actuation, said rods (30, 30') being secured to an axial slide (50) extending out of said body (10), said axial slide being axially displaceable with respect to said body (10) between a loaded position, in which said actuating springs (35, 35') are loaded, and an unloaded position after actuation, the user manually returning said slide to the loaded position after each actuation in order to reload said actuating springs (35, 35').

2. The device as claimed in claim 1, in which said receiving means comprise a window (13, 14) provided for each reservoir (20, 20') in a side wall of said body (10).

3. The device as claimed in claim 1 or 2, in which said first seal (22) is a membrane.

4. The device as claimed in any one of the preceding claims, in which said second sealing closure (23) is a plug, especially produced from an elastomer.

5. The device as claimed in any one of the preceding claims, in which each reservoir (20, 20') contains a different fluid product, the two different fluids being mixed upon actuation in order to be dispensed together.

6. The device as claimed in any one of claims 1 to 4, wherein each reservoir contains a fluid cleaning product and/or decontaminating product in order to form a cleaning reservoir.

7. The device as claimed in any one of the preceding claims, in which said actuation means (40) comprise a lateral actuation element which can be displaced in a direction which is different from the direction of displacement of said dispensing means (30, 30').

8. The device as claimed in any one of the preceding claims, in which said reservoir opening means (11) comprise means, such as a needle, for piercing said first sealing closure (22) of each reservoir (20, 20').

9. The device as claimed in claim 8, in which each needle (11) is associated with a needle spring (12) which biases a respective reservoir (20, 20') away from said needle (11) when it is installed in said body (10).

10. The device as claimed in any one of the preceding claims, in which said body (10), said dispensing means (30, 30'; 35, 35'), said actuating means (40) and said opening means (11) are assembled in order to form a unit, said reservoirs (20, 20') being installed in this unit after filling and sealing.

11. The device as claimed in any one of the preceding claims, in which said receiving means (13, 14) comprise a removable cover (9).
